# FASCICULE DE BREVET EUROPEEN

(11) **EP 3 927 309 B1**
(45) Date de publication et mention de la délivrance du brevet: **12.03.2025**
(21) Numéro de dépôt: 20710264.1
(22) Date de dépôt: 21.02.2020
(51) Int. Cl.: A61K 8/49, A61Q 19/00

(54) **UTILISATION DE L'EVODONE OU DE L'UN DE SES DÉRIVÉS COMME AGENT RAFRAÎCHISSANT**
VERWENDUNG VON EVODON ODER EINEM DERIVAT DAVON ALS KÜHLMITTEL
USE OF EVODONE OR A DERIVATIVE THEREOF AS A COOLING AGENT

(30) Priorité: 22.02.2019 FR 1901805
(43) Date de publication de la demande: 29.12.2021
(73) Titulaire: Robertet S.A., 06130 Grasse (FR)
(72) Inventeur: PEGARD, Anthony, 06130 GRASSE (FR); LAVOINE, Sophie, 06370 MOUANS SARTOUX (FR); HUMBERT, Marina, 06130 GRASSE (FR)
(74) Mandataire: Germain Maureau
(86) Numéro de dépôt international: PCT/FR2020/050326
(87) Numéro de publication internationale: WO 2020/169935

(56) Documents cités:
- WO-A1-2016/097496
- WO-A1-2017/106279
- US-A1- 2007 274 928

## Description

La présente invention a pour objet l'utilisation de l'evodone ou de l'un de ses dérivés comme agent rafraîchissant.

Dans l'ensemble du corps humain, et notamment sur la peau ou les muqueuses buccales, différents récepteurs permettant de percevoir diverses sensations. Les nocirécepteurs permettent par exemple de percevoir la douleur.

Parmi les nocirécepteurs, il existe dans la peau des terminaisons nerveuses libres, proches de capillaires sanguins, sensibles au froid ou au chaud : il s'agit des thermorécepteurs ou TRP (Transient Receptor Potential), des canaux ioniques qui s'ouvrent au-delà ou en dessous d'un seuil de température qui dépend du récepteur.

La famille des récepteurs TRP comprend plus de 30 canaux à cations et peut être divisée en sept sous-familles principales. Les canaux TRP et les récepteurs apparentés à TRP suggèrent une réponse sensorielle à la totalité des expositions thermiques, en répondant sélectivement à des températures seuils s'étalant de la chaleur nocive au froid nocif ainsi qu'à certains produits chimiques reproduisant ces sensations.

Les récepteurs au chaud, liés à des fibres amyéliniques de type C, sont plus profonds dans le derme. Les terminaisons nerveuses de la peau sensibles à la chaleur commencent à émettre des potentiels d'action lorsqu'elles sont soumises à une température d'environ 30°C. Plus la température augmente, plus la fréquence des potentiels formés augmente. Cependant, au-delà de 45°C, cette fréquence décroît (comme si le stimulus était plus faible). Par contre, les nocicepteurs, ces fibres sensibles à la douleur, commencent à émettre des potentiels d'action à partir de cette température.

Les récepteurs au froid, liés à des fibres myéliniques fines, sont superficiels, localisés dans l'épiderme. Les terminaisons nerveuses sensibles au froid commencent à émettre des potentiels d'action au voisinage de 35°C. La fréquence de ces potentiels augmente lorsque la température baisse (elle atteint son maximum vers 25°C puis décroît jusqu'à 10°C).

Il existe deux gènes, le TRPM8 et le TRPA1, connus pour être des canaux TRP sensibles au froid. En particulier, il est connu que le TRPM8, également appelé CMR1 (Cold and Menthol Receptor 1), est stimulé par des basses températures ainsi que par des composés dits « refroidissants ».

Des composés « refroidissants » permettant d'obtenir un effet thermique froid sont très souvent utilisés dans les produits cosmétiques, les produits d'hygiène ou de toilettes, ou encore dans les produits alimentaires, afin de simuler un effet rafraîchissant. L'utilisation de tels composés ou « agents rafraîchissants » permet notamment de renforcer la valence fraîcheur, l'effet tonifiant ou la sensation d'hydratation, permettant ainsi de compléter et/ou amplifier l'efficacité des produits dans lesquels ils sont incorporés.

Parmi les agents rafraîchissants les plus communément utilisés, on peut notamment citer le menthol ou encore l'iciline, la menthone, l'eucalyptol, le géraniol et le linalool. Ces agents ont un effet stimulant sur le canal TRPM8 ce qui déclenche cette sensation de fraîcheur.

L'utilité de ces agents rafraîchissants, et notamment du menthol et de ses dérivés, est toutefois limitée par ses actions multimodales sur les processus sensoriels, notamment du fait de son odeur, de la sévérité de son goût. Les effets désagréables du menthol peuvent être facilement ressentis, par exemple, lorsque des pommades contenant du menthol sont amenées près de la surface des yeux. Les vapeurs de menthol blessent l'œil et provoquent des larmoiements.

Ainsi, il demeure nécessaire à la date de la présente invention d'identifier de nouveaux composés susceptibles de stimuler le canal TRPM8 et donc être utilisés comme agents rafraîchissants dans les produits cosmétiques, les produits d'hygiène ou de toilette, ou encore dans les produits alimentaires, mais ne présentant pas les mêmes désavantages que le menthol en termes d'odeur ou de goût.

L'evodone, ou 6-diméthyl-6,7-dihydro-5H-1-benzofuran-4-one, peut être synthétisé ou isolé à partir d'extraits obtenus à partir des parties aériennes de la plante Euodia suaveolens Scheff. Comme l'indiquent les demandes de brevet internationales WO- A 2010/103207 et WO-A-2016/097496, l'evodone et ses proches dérivés sont notamment connus pour leurs propriétés répulsives contre les insectes, notamment les moustiques.

En revanche, l'éventuel effet de l'evodone ou de se dérivés sur le canal TRPM8 ou leur utilisation comme agent rafraîchissant n'a jamais été décrit à la date de la présente invention.

Le documeent WO 2017/106279 décrit l'utilisation de dérivés du menthol capables de moduler le canal TRPM8 et leur utilisation comme agent rafraîchissant. La demande de brevet US 2007/0274928 décrit l'utilisation de dérivés cycliques tels que les dérivés perhydrobenzofuranes pour leur effet rafraichissant. Cependant ces documents ne décrivent pas que des dérivés du benzofuranone telle que l'evodone pourraient posséder de telles propriétés.

Or, il a maintenant été trouvé, de façon tout à fait surprenante, que l'evodone et ses proches dérivés permettent de stimuler le canal TRPM8 et possèdent donc des propriétés rafraîchissantes.

La présente invention a donc pour objet l'utilisation comme agent rafraîchissant d'un composé de formule générale (I) 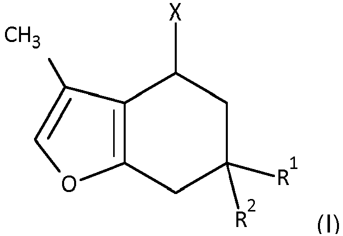 dans laquelle :
- R¹ et R² sont choisis indépendamment l'un de l'autre comme étant un atome d'hydrogène ou un groupe C₁-C₆-alkyle ; et
- X représente =O.

Les composés de formule (I) selon la présente invention peuvent être utilisés comme agents rafraîchissants dans les produits cosmétiques, les produits d'hygiène ou de toilettes, ou encore dans les produits alimentaires, avec des effets comparables à celui du menthol ou ses dérivés, et ne présentent pas les mêmes désavantages que le menthol en termes d'odeur ou de goût.

Dans le cadre de la présente invention :
- « evodone » désigne le 6-diméthyl-6,7-dihydro-5H-1-benzofuran-4-one dont le numéro CAS est 529-63-5 ;
- on entend par « C₁-C₆-alkyle » une chaîne hydrocarbonée saturée, linéaire ou ramifiée, et comportant de 1 à 6 atomes de carbone, notamment le groupe méthyle, ou éthyle ; et
- on entend par « agent rafraîchissant » tout composé susceptible de provoquer un effet thermique froid lorsqu'il est appliqué sur la peau ou introduits dans la cavité buccale, notamment en activant les canaux TRP sensibles au froid tels que le TRPM8 ou le TRPA1.

La présente invention a donc pour objet l'utilisation d'un composé de formule (I) tel que défini précédemment comme agent rafraîchissant. De préférence, la présente invention a pour objet l'utilisation comme agent rafraîchissant d'un composé de formule générale (I) dans lequel R¹ et R² sont choisis indépendamment l'un de l'autre comme étant un atome d'hydrogène ou un groupe -CH₃, -C₂H₅ ou -(CH₂)₂CH₃; de préférence encore R¹ et R² sont identiques et sont choisis comme étant un atome d'hydrogène.

De façon tout à fait préférée, la présente invention a pour objet l'utilisation de l'evodone comme agent rafraîchissant.

Les composés de formule (I) décrits précédemment peuvent être préparés par tout procédé connu de l'homme du métier.

Les composés de formule (I) décrits dans le cadre de la présente invention peuvent donc être utilisés comme agent rafraîchissant dans les produits cosmétiques, les produits d'hygiène ou de toilette, ou encore dans les produits alimentaires. Pour ce faire, ces composés peuvent être inclus dans une composition pouvant être formulée sous toute forme galénique appropriée à son administration.

Ainsi, les compositions comprenant l'agent rafraîchissant défini dans le cadre de la présente invention peuvent être formulées sous forme de crème, gel, lotion, mousse, sérum, lait, émulsion huile dans eau ou eau dans huile, solution, onguent, pulvérisateur, lotion après-rasage, savon, shampoing, poudre, bâton, crayon et stick pour maquillage, fond de teint, boisson, bonbon et chewing-gum.

Ces compositions contiennent l'agent rafraîchissant selon de la présente invention à des teneurs allant de 0,005% à 75% en poids total de la composition, préférentiellement de 0,01% à 25%, préférentiellement encore de 0,1% à 5%.

Pour la préparation de ces compositions, l'agent rafraîchissant utilisé dans le cadre de la présente invention est mélangé aux excipients classiquement utilisés dans le domaine cosmétique ou nutraceutique.

Les compositions comprenant l'extrait utilisé dans le cadre de la présente invention peuvent prendre la forme d'une crème dans laquelle l'agent rafraîchissant utilisé dans le cadre de la présente invention est associé aux excipients couramment utilisés en cosmétologie.

Ces compositions peuvent également prendre la forme de gels dans les excipients appropriés tels que les esters de cellulose ou d'autres agents gélifiants, tels que le carbopol, le sepinov (polyacrylate), la gomme guar, etc.

Ces compositions peuvent aussi prendre la forme d'une lotion ou d'une solution dans lesquelles l'agent rafraîchissant utilisé dans le cadre de la présente invention est sous forme encapsulée. Les microsphères peuvent par exemple être constituées de corps gras, d'agar et d'eau. L'agent rafraîchissant utilisé dans le cadre de la présente invention peut être incorporé dans des vecteurs de type liposomes, glycosphères, cyclodextrines, dans des chylomicrons, des macro-, micro-, nano-particules ainsi que les macro-, micro- et nanocapsules et aussi être absorbé sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.

Les compositions comprenant l'agent rafraîchissant utilisé dans le cadre de la présente invention peuvent aussi contenir des additifs ou des adjuvants usuels en cosmétologie, comme par exemple des agents antimicrobiens ou des parfums mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

Les compositions comprenant l'agent rafraîchissant utilisé dans le cadre de la présente invention peuvent aussi comprendre d'autres principes actifs rafraîchissants tels que le citriodiol ou le poivre de cubebe et/ou des actifs complémentaires choisis pour leur action, par exemple pour leur effet nutritionnel, l'effet amincissant, l'effet anti-cellulite, l'effet raffermissant, l'effet hydratant, l'effet anti-âge, l'activité antimicrobienne, l'activité anti-oxydante, l'activité anti-radicalaire, l'effet cicatrisant, l'effet tenseur, l'effet anti-ride, l'activité chélatante, l'activité complexante et séquestrante, l'effet apaisant, l'effet anti-cernes, l'effet anti-rougeurs, l'activité émolliente, l'effet démêlant capillaire, l'activité anti-pelliculaire, l'effet stimulant de la repousse du cheveu, l'effet inhibant la chute du cheveu, l'effet gainant capillaire, l'activité épilatoire, l'activité limitant la repousse du poil, l'activité participant au renouvellement cellulaire, l'activité modulant la réponse inflammatoire, l'activité participant au maintien de l'ovale du visage, mais également la protection solaire, l'activité anti-irritante, la nutrition cellulaire, la respiration cellulaire, les traitements anti-séborrhéiques, la tonicité cutanée, la protection du cheveu.

Lorsque les compositions comprenant l'agent rafraîchissant utilisé dans le cadre de la présente invention contiennent des principes actifs complémentaires, ceux-ci sont généralement présents dans la composition à une concentration suffisamment élevée pour qu'ils puissent exercer leur activité.

Les compositions comprenant l'agent rafraîchissant utilisé dans le cadre de la présente invention sont très bien tolérées, elles ne présentent aucune toxicité et leur application sur la peau ou leur introduction dans la cavité buccale, même pour des périodes de temps prolongées, n'implique aucun effet systématique.

La présente invention est illustrée de manière non limitative par les exemples suivants.

### Exemple 1 - Crème contenant l'evodone

Des crèmes (émulsion eau dans huile) contenant respectivement :
- 1% d'evodone - crème 1;
- 1% de Frescolat ML (i.e. menthyl lactate) - crème 2 ;
- 1% d'Eucalydiol (i.e. citriodiol) - crème 3 ;
- 1% d'extrait CO₂ de cucebe (30% de cubelol) - crème 4 ;
sont préparées.

Pour mesurer l'effet rafraîchissant, on applique 100 mg de l'une des crèmes 1 à 4 ou d'un placebo sur la face interne des deux avant-bras de personnes ayant accepté de tester le produit. Au bout d'une minute, il est demandé à chaque participant d'évaluer l'effet rafraîchissant sur une échelle de 0 à 3 (i.e. score), 3 étant l'effet maximal.

Chaque crème (y compris le placebo) est appliquée de manière aléatoire et à l'aveugle sur les avant-bras de 17 personnes.

Les résultats obtenus sont rapportés dans le Tableau 1 suivant.

**Tableau 1 - Pouvoir rafraîchissant des crèmes 1 à 4**

| **Crème testée** | **Pouvoir rafraîchissant (obtenu en ajoutant les scores)** |
|---|---|
| Crème 1 | 30 |
| Crème 2 | 25 |
| Crème 3 | 15 (p<0,05) |
| Crème 4 | 30 |

On note que l'evodone a un effet rafraîchissant similaire à celui que peuvent apporter des molécules telles que le citriodiol ou le cubebol se trouvant dans l'extrait CO2 de poivre de cubebe. En outre, l'effet rafraîchissant est significativement supérieur à celui obtenu avec le menthyl lactate (i.e. Frescolat ML - référence commerciale).

### Exemple 2 - Crème contenant l'evodone

### 2.1 - Protocole expérimental

Pour cette étude, l'effet rafraîchissant de 5 compositions contenant différents agents rafraîchissants est évaluée *in vivo* selon le protocole suivant.

22 sujets féminins âgés de 18 à 63 ans ont été sélectionnés. Sur le dos de chaque sujet, entre les deux scapulas, on délimite six zones de 3cm x 2,5cm. Sur 5 zones choisies à chaque fois au hasard on applique 15mg des compositions à tester. Rien n'est appliqué sur la sixième zone (i.e. contrôle).

A compter de l'application des crèmes (T0), la température cutanée des différentes zones est mesurée sur chaque sujet grâce à une caméra thermique (Thermovision SC6000HS (Flirsystems) - résolution 640x512 - fréquence 125 Hz) aux temps suivants : T0, T0+15s, T0+30s, T0+45s, T0+1min, T0+1min30s, T0+2min, T0+3min, T0+4min, T0+5min, T0+6min, T0+8min et T0+10min. Les mesures sont effectuées à 21°C+/-1,5°C, le sujet étant allongé sur le ventre et immobile. Les températures sont mesurées avec une précision de l'ordre de 0,10°C.

Un test de variance (ANOVA) est utilisé pour compiler et valider les résultats. La normalité des résidus de l'ANOVA est vérifiée par le test de Shapiro-Wilk avec un niveau de significativité fixé à 1%, et l'homogénéité des variances sur les résidus est vérifiée par un test de Levene à 5%.

Si ces deux conditions sont remplies, l'analyse statistique permettant de comparer les paramètres mesurés aux différents temps est réalisée par un test d'analyse de variance (ANOVA) à un facteur sur mesures répétées. Le niveau de significativité est fixé à 5%.

Si l'une des deux conditions n'est pas remplie, l'analyse statistique est effectuée par une ANOVA sur les données transformées en rangs, à un seuil de 5%.

En cas de différence significative dans l'ANOVA, un test de Dunnett à 5% est effectué pour comparer tous les temps cinétiques au T0.

### 2.2 - Compositions utilisées

Les crèmes 1 à 4 décrites dans l'exemple 1 ont été testées. En outre, la crème 5 contenant 33,3% d'evodone / 33,3% d'eucalydiol / 33,3% d'extrait de cubèbe (30% de cubèbol) a également été testée.

### 2.3 - Résultats expérimentaux

Les résultats expérimentaux obtenus sont rapportés dans le Tableau 2 ci-dessous.

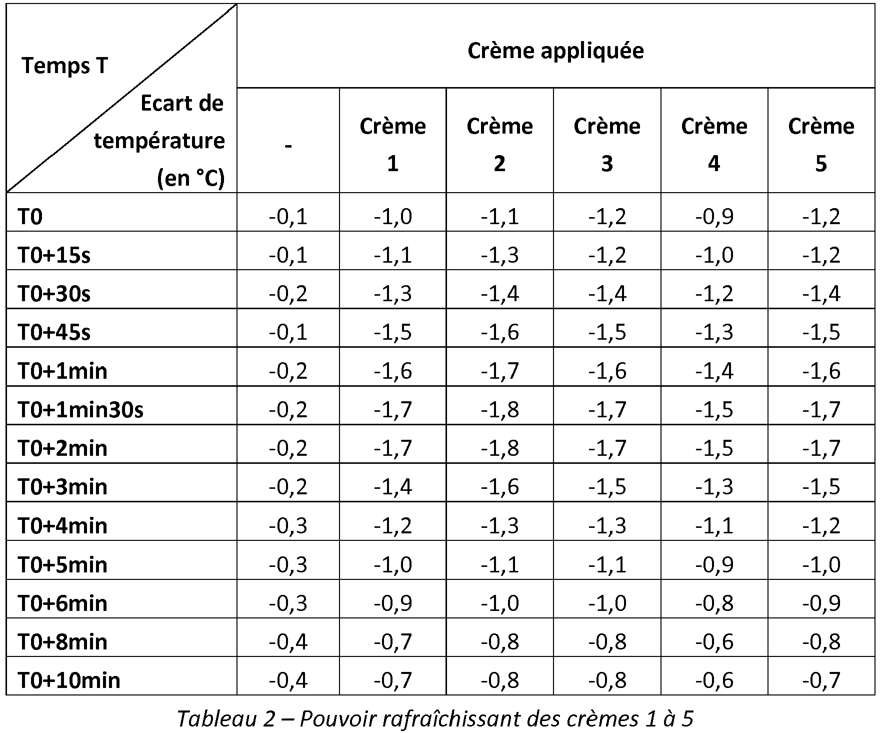

### 2.4 - Conclusion

On note que l'evodone a un effet rafraîchissant similaire à celui que peuvent apporter des molécules telles que le menthyl lactate (i.e. Frescolat ML - référence commerciale), le citriodiol ou le cubebol se trouvant dans l'extrait CO₂ de poivre de cubebe.

## Revendications

1. Utilisation comme agent rafraîchissant d'un composé de formule générale (I) 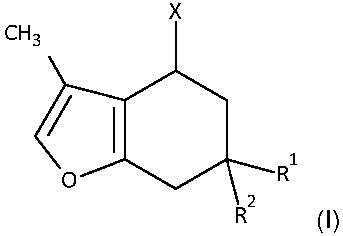 dans laquelle :
- R¹ et R² sont choisis indépendamment l'un de l'autre comme étant un atome d'hydrogène ou un groupe C₁-C₆-alkyle ; et
- X représente =O.

2. Utilisation comme agent rafraîchissant d'un composé de formule générale (I) selon la revendication 1, **caractérisé en ce que** R¹ et R² sont choisis indépendamment l'un de l'autre comme étant un atome d'hydrogène ou un groupe -CH₃, -C₂H₅ ou - (CH₂)₂CH₃.

3. Utilisation comme agent rafraîchissant d'un composé de formule générale (I) selon la revendication 1 ou 2, **caractérisé en ce que** R¹ et R² sont identiques et sont choisis comme étant un atome d'hydrogène.

4. Utilisation comme agent rafraîchissant d'un composé de formule générale (I) selon l'une quelconque des revendications 1 à 3, **caractérisée en ce que** le composé de formule générale (I) est l'evodone.

## Patentansprüche

1. Verwendung einer Verbindung der allgemeinen Formel (I) als kühlendes Mittel, 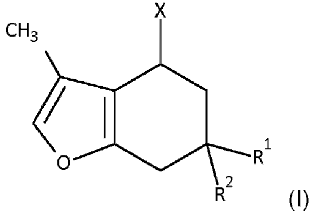 wobei:
- R¹ und R² unabhängig voneinander als Wasserstoffatom oder eine C₁-C₆-Alkylgruppe ausgewählt sind; und
- X für =0 steht.

2. Verwendung einer Verbindung der allgemeinen Formel (I) als kühlendes Mittel nach Anspruch 1, **dadurch gekennzeichnet, dass** R¹ und R² unabhängig voneinander als Wasserstoffatom oder -CH₃-, -C₂H₅- oder - (CH₂ )₂CH₃-Gruppe ausgewählt sind.

3. Verwendung einer Verbindung der allgemeinen Formel (I) als kühlendes Mittel nach Anspruch 1 oder 2, **dadurch gekennzeichnet, dass** R¹ und R² identisch sind und als Wasserstoffatom ausgewählt sind.

4. Verwendung einer Verbindung der allgemeinen Formel (I) als kühlendes Mittel nach einem der Ansprüche 1 bis 3, **dadurch gekennzeichnet, dass** die Verbindung der allgemeinen Formel (I) Evodon ist.

## Claims

1. Use as a cooling agent of a compound of general formula (I) 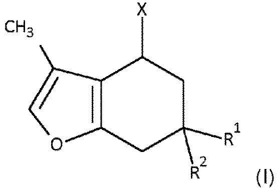 in which:
- R¹ et R² are independently of each other selected as being a hydrogen atom or a C₁-C₆-alkyl group; and
- X represents =O.

2. Use as a cooling agent of a compound of general formula (I) according to claim 1, **characterized in that** R¹ and R² are selected independently of one another as being a hydrogen atom or a group -CH₃, -C₂H₅ or -(CH₂)₂CH₃.

3. Use as a cooling agent of a compound of general formula (I) according to claim 1 or 2, **characterized in that** R¹ and R² are identical and are selected as being a hydrogen atom.

4. Use as a cooling agent of a compound of general formula (|) according to any one of claims 1 to 3, **characterized in that** the compound of general formula (1) is evodone.
